# EUROPÄISCHE PATENTANMELDUNG

(11) **EP 4 014 855 A1**
(43) Veröffentlichungstag der Anmeldung: **22.06.2022**
(21) Anmeldenummer: 20215732.7
(22) Anmeldetag: 18.12.2020
(51) Int. Cl.: A61B 5/055, A61B 5/00

(54) **MAGNETRESONANZVORRICHTUNG MIT EINER PATIENTENLAGERUNGSVORRICHTUNG**

(71) Anmelder: Siemens Healthcare GmbH, 91052 Erlangen (DE)
(72) Erfinder: Kurth, Rainer, 91056 Erlangen (DE); Zink, Stephan, 90451 Nürnberg (DE)

(57) **Zusammenfassung**

Eine Magnetresonanzvorrichtung mit einer Scannereinheit, einem von der Scannereinheit zumindest teilweise umgebenen Patientenaufnahmebereich und einer Patientenlagerungsvorrichtung weist einen innerhalb des Patientenaufnahmebereichs bewegbaren Patiententisch auf. Diese besitzt eine Vertikaleinstelleinheit zu einer Einstellung einer Vertikalposition des Patiententischs innerhalb des Patientenaufnahmebereichs, wobei die Vertikaleinstelleinheit zumindest zwei unterschiedliche Vertikalpositionen für eine Positionierung des Patiententischs innerhalb des Patientenaufnahmebereiches umfasst.

## Beschreibung

Die vorliegende Erfindung betrifft eine Magnetresonanzvorrichtung mit einer Scannereinheit, einem von der Scannereinheit zumindest teilweise umgebenen Patientenaufnahmebereich und einer Patientenlagerungsvorrichtung, die einen innerhalb des Patientenaufnahmebereichs bewegbaren Patiententisch aufweist.

Magnetresonanzvorrichtung weisen für gewöhnlich einen Patientenaufnahmebereich auf, in dem ein Patient für eine Untersuchung, insbesondere eine Magnetresonanzuntersuchung, gelagert und/oder positioniert wird. Bevorzugt soll hierbei der Patient, insbesondere der zu untersuchende Bereich des Patienten, in einem Isozentrum der Magnetresonanzvorrichtung positioniert werden, um ideale Bedingungen für eine Erfassung von Magnetresonanzdaten zu erhalten. Hierzu ist der Patient auf einem Patiententisch einer Patientenlagerungsvorrichtung positioniert. Insbesondere bei zylinderförmig ausgebildeten Patientenaufnahmebereichen von Magnetresonanzvorrichtungen weist der Patiententisch innerhalb des Patientenaufnahmebereichs in vertikaler Richtung eine einzige Position auf. Diese Position ermöglicht für einen Durchschnittspatienten eine im Wesentlichen optimale Positionierung innerhalb des Patientenaufnahmebereichs, insbesondere hinsichtlich des Isozentrums der Magnetresonanzvorrichtung. Jedoch stellt dies Position in vertikaler Richtung für Patienten, die aufgrund ihrer Anatomie stark von einem durchschnittlichen Patienten abweichen, einen Kompromiss in der Positionierung dar. Die Position des Patiententisch ist in vertikaler Richtung auch nicht verstellbar und/oder einstellbar ausgebildet. Dies führt dazu, dass unabhängig von einer Patientenanatomie und/oder einer Art der Magnetresonanzuntersuchung der Patiententisch in vertikaler Richtung immer die gleiche Position einnimmt.

Zwar kann bei einem erhöhten Platzbedarf in vertikaler Richtung die an und/oder in dem Patiententisch angeordnete Spine-Hochfrequenzspule von dem Patiententisch für die anstehende Untersuchung des Patienten entfernt werden. Jedoch steht diese Hochfrequenzspule dann auch nicht mehr für eine Datenerfassung zur Verfügung, so dass hierdurch die Bilddatenqualität negativ beeinflusst werden kann. Bei kleinen Patienten, wie beispielsweise Kindern, ist es bekannt, dass für eine optimale Positionierung Lagerungskissen verwendet werden können, um einen zu untersuchenden Bereich des Patienten, insbesondere eines Kindes, im Isozentrum der Magnetresonanzvorrichtung anzuordnen und/oder zu positionieren. Jedoch kann dies bei einer Untersuchung der Wirbelsäule zu einer erheblichen Signalschwächung führen und damit zu einer Beeinträchtigung einer Bildqualität in den erfassten Magnetresonanzbilddaten.

Der vorliegenden Erfindung liegt insbesondere die Aufgabe zugrunde, eine Position des Patiententischs in vertikaler Richtung innerhalb des Patientenaufnahmebereichs einstellbar zu gestalten. Die Aufgabe wird durch die Merkmale des unabhängigen Anspruchs gelöst. Vorteilhafte Ausgestaltungen sind in den Unteransprüchen beschrieben.

Die Erfindung geht aus von einer Magnetresonanzvorrichtung mit einer Scannereinheit, einem von der Scannereinheit zumindest teilweise umgebenen Patientenaufnahmebereich und einer Patientenlagerungsvorrichtung, die einen innerhalb des Patientenaufnahmebereichs bewegbaren Patiententisch aufweist. Erfindungsgemäß weist die Magnetresonanzvorrichtung eine Vertikaleinstelleinheit zu einer Einstellung einer Vertikalposition des Patiententischs innerhalb des Patientenaufnahmebereichs auf, wobei die Vertikaleinstelleinheit zumindest zwei unterschiedliche Vertikalpositionen für eine Positionierung des Patiententischs innerhalb des Patientenaufnahmebereiches umfasst.

Die Magnetresonanzvorrichtung umfasst bevorzugt eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten ausgelegt und/oder ausgebildet ist. Die Magnetresonanzvorrichtung umfasst hierzu die Scannereinheit. Die Scannereinheit der Magnetresonanzvorrichtung umfasst bevorzugt eine Detektoreinheit, insbesondere eine Magneteinheit, zur Erfassung der medizinischen und/oder diagnostischen Bilddaten. Bevorzugt umfasst hierbei die Scannereinheit, insbesondere die Magneteinheit, einen Grundmagneten, ein Gradienten-System und eine Hochfrequenzantenneneinheit. Die Hochfrequenzantenneneinheit ist fest innerhalb der Scannereinheit angeordnet und zur Aussendung eine Anregungspulses ausgelegt und/oder ausgebildet. Des Weiteren weist die Magnetresonanzvorrichtung zumindest eine lokalen Hochfrequenzspule auf, die zu einem Empfangen eines Magnetresonanzsignals ausgebildet ist. Hierzu wird die lokale Hochfrequenzspule um den zu untersuchenden Bereich des Patienten angeordnet und/oder angelegt. Bevorzugt sind die einzelnen lokalen Hochfrequenzspulen speziell auf einen Untersuchungsbereich von Patienten ausgelegt und/oder ausgebildet, wie beispielsweise eine Kopfhochfrequenzspule oder eine Kniehochfrequenzspule usw.

Der Grundmagnet ist zur Erzeugung eines homogenen Grundmagnetfelds mit einer definierten Magnetfeldstärke, wie beispielsweise mit einer Magnetfeldstärke von 3 T oder 1,5 T usw., ausgebildet. Insbesondere ist der Grundmagnet zur Erzeugung eines starken und konstanten Grundmagnetfelds ausgebildet. Das homogene Grundmagnetfeld ist bevorzugt innerhalb eines Patientenaufnahmebereichs der Magnetresonanzvorrichtung angeordnet und/oder vorzufinden. Das Gradienten-System ist zu einer Erzeugung von Magnetfeldgradienten, die für eine Ortskodierung während einer Bildgebung verwendet werden, ausgebildet.

Der Patientenaufnahmebereich ist zu einer Aufnahme des Patienten, insbesondere des zu untersuchenden Bereichs des Patienten, für eine medizinische Magnetresonanzuntersuchung ausgelegt und/oder ausgebildet. Beispielsweise ist hierzu der Patientenaufnahmebereich zylinderförmig ausgebildet und/oder zylinderförmig von der Scannereinheit, insbesondere der Magneteinheit, der Magnetresonanzvorrichtung umgeben. Innerhalb des Patientenaufnahmebereichs ist bevorzugt ein Field of View (FOV) und/oder ein Isozentrum der Magnetresonanzvorrichtung angeordnet. Das FOV umfasst bevorzugt einen Erfassungsbereich der Magnetresonanzvorrichtung, innerhalb dessen die Bedingungen für eine Erfassung von medizinischen Bilddaten, insbesondere von Magnetresonanzbilddaten, innerhalb des Patientenaufnahmebereichs vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten aufweist. Insbesondere umfasst das Isozentrum den homogensten Magnetfeldbereich innerhalb der Magnetresonanzvorrichtung.

Für eine Positionierung des Patienten, insbesondere des zu untersuchenden Bereichs des Patienten, innerhalb des Patientenaufnahmebereich weist die Magnetresonanzvorrichtung die Patientenlagerungsvorrichtung auf. Die Patientenlagerungsvorrichtung ist zu einer Lagerung des Patienten ausgebildet. Die Patientenlagerungsvorrichtung weist bevorzugt einen bewegbaren Patiententisch auf, der insbesondere innerhalb des Patientenaufnahmebereichs der Magnetresonanzvorrichtung bewegbar ausgebildet ist. Für eine Magnetresonanzuntersuchung wird der Patient zunächst auf dem Patiententisch der Patientenlagerungsvorrichtung positioniert und anschließend der Patiententisch zusammen mit dem Patienten in den Patientenaufnahmebereich eingefahren, bis der zu untersuchende Bereich des Patienten innerhalb des Isozentrums positioniert ist.

Die Vertikaleinstelleinheit der Magnetresonanzvorrichtung ist dazu ausgebildet, eine vertikale Position des Patiententischs einzustellen. Dabei kann die Vertikaleinstelleinheit ein Vertikaleinstellelement oder auch mehrere Vertikaleinstellelemente aufweisen, die an der Patientenlagerungsvorrichtung, wie beispielsweise am Patiententisch, und/oder innerhalb des Patientenaufnahmebereichs angeordnet sein können. Die Vertikaleinstelleinheit, insbesondere die einzelnen Vertikaleinstellelemente der Vertikaleinstelleinheit, sind dazu ausgebildet, zumindest zwei unterschiedliche Vertikalpositionen für die Positionierung des Patiententischs in vertikaler Richtung zur Verfügung zu stellen und/oder eine Position des Patiententischs in vertikaler Richtung in eine der zumindest zwei unterschiedlichen Vertikalpositionen einzustellen. Dabei kann die Vertikaleinstelleinheit, insbesondere die einzelnen Vertikaleinstellelemente der Vertikaleinstelleinheit, dazu ausgebildet sein, dass die Einstellung zwischen den zumindest zwei unterschiedlichen Vertikalpositionen über unterschiedliche Positionsstufen, insbesondere definierte und/oder feststehende Positionsstufen, erfolgen kann. Alternativ hierzu kann die Vertikaleinstelleinheit auch einen Einstellbereich, insbesondere einen Vertikaleinstellbereich, umfassen, wobei die zumindest zwei unterschiedlichen Vertikalpositionen beliebig, insbesondere von einem Benutzer frei wählbar, innerhalb des Einstellbereichs einstellbar sind.

Bevorzugt weist die Vertikaleinstelleinheit zumindest drei unterschiedliche Vertikalpositionen für eine Positionierung des Patiententischs in vertikaler Richtung innerhalb des Patientenaufnahmebereichs auf. Beispielsweise kann hierbei eine mittlere Vertikalposition der drei Vertikalpositionen eine Grundposition des Patiententischs in vertikaler Richtung umfassten, wobei die mittlere Vertikalposition und/oder die Grundposition des Patiententisch in vertikaler Richtung insbesondere für Patienten mit einer durchschnittlichen Anatomie verwendet wird. Eine untere Vertikalposition der drei Vertikalpositionen kann insbesondere für Patienten mit einer überdurchschnittlichen Anatomie, wie beispielweise für adipös veranlagte Patienten, und/oder für Patienten mit einem erhöhten Platzbedarf verwendet werden, da hier mehr verfügbarer Raum zwischen dem Patiententisch, insbesondere einem Lagerungsbereich des Patiententischs zur Lagerung des Patienten, und einer den Patientenaufnahmebereich umgebenden Umhausung vorhanden ist. Eine obere Vertikalposition der drei Vertikalpositionen kann insbesondere für Patienten mit einer unterdurchschnittlichen Anatomie, wie beispielweise für Kinder, verwendet werden, um den zu untersuchenden Bereich des Patienten innerhalb des Isozentrums der Magnetresonanzvorrichtung zu positionieren.

Bevorzugt weist der Patientenaufnahmebereich für eine Einstellung einer Vertikalposition des Patiententischs aus mehreren zur Verfügung stehenden Vertikalpositionen einen Durchmesser und/oder eine Quererstreckung von größer 50 cm auf. Besonders vorteilhaft weist Patientenaufnahmebereich einen Durchmesser und/oder eine Quererstreckung von größer 60 cm auf. Besonders vorteilhaft weist Patientenaufnahmebereich einen Durchmesser und/oder eine Quererstreckung von größer 70 cm auf. Besonders vorteilhaft weist Patientenaufnahmebereich einen Durchmesser und/oder eine Quererstreckung von größer 75 cm auf. Besonders vorteilhaft weist Patientenaufnahmebereich einen Durchmesser und/oder eine Quererstreckung von 80 cm auf.

Die Erfindung weist den Vorteil auf, dass eine Positionierung des Patiententischs in vertikaler Richtung innerhalb des Patientenaufnahmebereichs möglich ist. Insbesondere kann hierbei die Positionierung des Patiententischs in vertikaler Richtung an eine Anatomie des Patienten und/oder an eine Art der Magnetresonanzuntersuchung angepasst werden. Insbesondere kann hierbei bei Magnetresonanzuntersuchung mit einem erhöhten Platzbedarf innerhalb des Patientenaufnahmebereichs der Patiententisch in eine niedrigere Vertikalposition gebracht und/oder gefahren werden und damit mehr Platz für den Patienten und/oder für Zusatzeinheiten auf dem Patiententisch zur Verfügung gestellt werden. Magnetresonanzuntersuchungen mit einem erhöhten Platzbedarf können beispielsweise Magnetresonanzuntersuchungen von korpulenten Patienten und/oder Magnetresonanzuntersuchungen, bei denen ein erhöhter Platzbedarf durch Anbringen von Zusatzeinheiten auf dem Patiententisch, wie beispielsweise Untersuchungen von einer Brust und/oder einem Knie und/oder einer Prostata von Patienten, benötigt wird, umfassen. Dabei kann insbesondere bei adipös veranlagten Patienten und/oder auch klaustrophobisch veranlagten Patienten mehr Platz während einer Magnetresonanzuntersuchung zur Verfügung gestellt werden.

Ein weitere Vorteil der Erfindung ist, dass der zu untersuchende Bereich eines Patienten in vertikaler Richtung gezielt innerhalb des Isozentrums für die anstehende Magnetresonanzuntersuchung angeordnet werden kann. Zudem kann auch bei Untersuchungen von Kindern und/oder sehr dünnen Patienten, bei denen der zu untersuchende Bereich in einer Normalposition des Patiententisches unterhalb des Isozentrums wäre, der Patiententisch in eine höhere Vertikalposition gebracht und/oder gefahren werden, um somit den zu untersuchenden Bereich des Patienten innerhalb des Isozentrums der Magnetresonanzvorrichtung zu positionieren.

In einer vorteilhaften Weiterbildung der erfindungsgemäßen Magnetresonanzvorrichtung kann es vorgesehen sein, dass die Vertikaleinstelleinheit zumindest ein Vertikaleinstellelement umfasst, dass innerhalb des Patientenaufnahmebereichs angeordnet ist. Dabei kann das zumindest eine Vertikaleinstellelement ein passives Vertikaleinstellelement umfassen, das zur Aufnahme des Patiententischs in einer Vertikalposition, beispielsweise in einer definierten Vertikalposition, ausgebildet ist. Zudem kann das zumindest eine Vertikaleinstellelement auch ein aktives Vertikaleinstellelement umfassen, das zur direkten Einstellung einer definierten Vertikalposition des Patiententischs ausgebildet ist. Unter einer Anordnung innerhalb des Patientenaufnahmebereichs des zumindest einen Vertikaleinstellelements soll hierbei insbesondere eine Anordnung des zumindest einen Vertikaleinstellelements an einer den Patientenaufnahmebereich umgebenden Umhausung verstanden werden. Durch die Anordnung von zumindest einem Vertikaleinstellelement der Vertikaleinstelleinheit innerhalb des Patientenaufnahmebereichs kann eine besonders kompakte Anordnung der Vertikaleinstelleinheit erreicht werden.

In einer vorteilhaften Weiterbildung der erfindungsgemäßen Magnetresonanzvorrichtung kann es vorgesehen sein, dass das zumindest eine Vertikaleinstellelement eine Seitenführung für den Patiententisch mit zwei unterschiedlichen Aufnahmebereichs zur Aufnahme des Patiententischs umfasst. Die Seitenführung für den Patiententisch ist bevorzugt innerhalb des Patientenaufnahmebereichs angeordnet und zur Führung des Patiententischs innerhalb des Patientenaufnahmebereichs ausgebildet. Hierbei verläuft die Seitenführung an einem Randbereich des Patientenaufnahmebereichs. Hierbei können die beiden Seitenführungen in eine Umhausung des Patientenaufnahmebereichs integriert sein. Bevorzugt sind zwei Seitenführungen zur Führung des Patiententischs innerhalb des Patientenaufnahmebereichs angeordnet, wobei die beiden Seitenführungen an unterschiedlichen, insbesondere sich gegenüberliegenden Seiten des Patientenaufnahmebereichs angeordnet sind. Zwischen den beiden Seitenführungen wird der Patiententisch der Patientenlagerungsvorrichtung innerhalb des Patientenaufnahmebereichs geführt.

Vorzugsweise weist jede der beiden Seitenführungen jeweils zumindest zwei unterschiedliche Aufnahmebereiche zur Aufnahme des Patiententischs auf und damit auch zumindest zwei unterschiedliche Vertikalpositionen für die Positionierung des Patiententischs, so dass für den Patiententisch zumindest zwei unterschiedliche Vertikalpositionen zur Verfügung stehen. Bevorzugt weist jeder der beiden Seitenführungen jeweils drei unterschiedliche Aufnahmebereichs zur Aufnahme des Patiententischs auf, so dass für den Patiententisch drei unterschiedliche Vertikalpositionen zur Einstellung einer Vertikalposition des Patiententischs zur Verfügung stehen. Die einzelnen Aufnahmebereichs können hierbei als Führungsschiene zur Führung und/oder Aufnahme des Patiententischs ausgebildet sein. Insbesondere unterscheiden sich die unterschiedlichen Aufnahmebereich hinsichtlich ihrer Anordnung in vertikaler Richtung an den Seitenführungen.

Ein Vorteil einer derartigen Ausgestaltung der Erfindung ist, dass eine konstruktiv einfache und besonders kostengünstige Vertikaleinstelleinheit zur Einstellung einer Position des Patiententischs bereitgestellt werden kann.

In einer vorteilhaften Weiterbildung der erfindungsgemäßen Magnetresonanzvorrichtung kann es vorgesehen sein, dass die Vertikaleinstelleinheit zumindest ein Vertikaleinstellelement umfasst, das an einer rückseitigen Öffnung des Patientenaufnahmebereichs zu einer Aufnahme des Patiententischs angeordnet ist. Insbesondere ist das Vertikaleinstellelement zur Abstützung eines zumindest teilweise schwebend gelagerten Patiententischs innerhalb des Patientenaufnahmebereichs ausgebildet. Hierbei ragt der zumindest teilweise schwebend gelagerte Patiententisch mit einem in Einfahrtrichtung vorderem Ende frei in den Patientenaufnahmebereich hinein und ist nur mit einem hinterem Ende an der Patientenlagerungsvorrichtung gelagert. Vorzugsweise ist das Vertikaleinstellelement vor einer Aufnahme und/oder Abstützung des frei in den Patientenaufnahmebereich ragenden Endes des Patiententischs auf eine Höhe und/oder Vertikalposition des Patiententischs eingestellt und/oder abgestimmt. Für die Abstützung des Patiententischs kann das Vertikaleinstellelement auch von der rückseitigen Öffnung aus in den Patientenaufnahmebereich hineinragen, so dass der Patiententisch bereits ab beispielsweise einer Mitte des Patientenaufnahmebereichs von dem Vertikaleinstellelement aufgenommen werden kann. Durch diese Ausgestaltung der Erfindung kann ein unerwünschtes Durchbiegen des Patiententischs verhindert werden.

In einer vorteilhaften Weiterbildung der erfindungsgemäßen Magnetresonanzvorrichtung kann es vorgesehen sein, dass das zumindest eine Vertikaleinstellelement ein aktives Vertikaleinstellelement aufweist zu einer direkten Einstellung einer Vertikalposition des Patiententischs innerhalb des Patientenaufnahmebereichs. Beispielsweise kann ein derartiges aktives Vertikaleinstellelement zu einer Einstellung und/oder eine Anpassung zumindest eines Führungselements, wie beispielsweise eine Führungsschiene, zur Führung des Patiententischs in vertikaler Richtung innerhalb des Patientenaufnahmebereichs ausgebildet sein. Vorzugsweise erfolgt eine vertikale Anpassung des zumindest einen Führungselements in Abhängigkeit von einer Vertikalposition und/oder einer Höhe des Patiententischs. Das aktive Vertikaleinstellelement kann beispielsweise eine Hubschere und/oder weitere, dem Fachmann als sinnvoll erscheinende aktive Vertikaleinstellelemente umfassen. Hierdurch eine einfache und direkte Einstellung einer Vertikalposition des Patiententischs erreicht werden.

In einer vorteilhaften Weiterbildung der erfindungsgemäßen Magnetresonanzvorrichtung kann es vorgesehen sein, dass die Vertikaleinstelleinheit zumindest ein Vertikaleinstellelement aufweist, das an der Patientenlagerungsvorrichtung angeordnet ist. Das zumindest eine Vertikaleinstellelement an der Patientenlagerungsvorrichtung umfasst bevorzugt ein aktives Vertikaleinstellelement zur direkten Einstellung einer Vertikalposition des Patiententischs. Dabei kann das zumindest eine aktive Vertikaleinstellelement zu einer Einstellung einer Vertikalposition des Patiententisch ausgebildet sein, wenn der Patiententisch sich noch in einer Ausgangsposition befindet. In der Ausgangsposition des Patiententisch befindet sich dieser noch komplett außerhalb des Patientenaufnahmebereichs. Zudem kann das zumindest eine aktive Vertikaleinstellelement auch zur einer Einstellung einer Vertikalposition des Patiententischs ausgebildet sein, wenn der Patiententisch sich bereits in einer In-Bore-Position befindet. In der In-Bore-Position des Patiententischs befindet sich der Patiententisch zumindest teilweise innerhalb des Patientenaufnahmebereichs. Diese Ausgestaltung der Erfindung weist den Vorteil auf, dass kein zusätzlicher Bauraum innerhalb des Patientenaufnahmebereichs zur Einstellung einer Vertikalposition des Patiententischs benötigt wird und damit ein vorteilhaftes Platzangebot für den Patienten während einer Magnetresonanzmessung zur Verfügung steht.

In einer vorteilhaften Weiterbildung der erfindungsgemäßen Magnetresonanzvorrichtung kann es vorgesehen sein, dass das zumindest eine Vertikaleinstellelement an einer Unterseite des Patiententischs angeordnet ist. Die Unterseite des Patiententisch umfasst eine einem Lagerungsbereich für den Patienten abgewandte Seite und/oder Oberfläche des Patiententischs. Diese Ausgestaltung ermöglicht eine besonders platzsparende Anordnung des Patiententischs. Zudem kann derart auch eine hohe Sicherheit für den Patienten erreicht werden, da ein Verletzung durch das zumindest eine Vertikaleinstellelement aufgrund der Anordnung des zumindest einen Vertikaleinstellelements an der Unterseite des Patiententischs vorteilhaft verhindert werden kann. Das zumindest eine Vertikaleinstellelement kann hierbei ein aktives Vertikaleinstellelement umfassen, wie beispielsweise zumindest eine an der Unterseite des Patiententischs angeordnete Hubschere und/oder weitere, dem Fachmann als sinnvoll erscheinende aktive Vertikaleinstellelemente umfassen.

In einer vorteilhaften Weiterbildung der erfindungsgemäßen Magnetresonanzvorrichtung kann es vorgesehen sein, dass die Vertikaleinstelleinheit zumindest ein Vertikaleinstellelement aufweist, wobei das zumindest eine Vertikaleinstellelement eine Hubschere umfasst. Hierdurch kann eine besonders kostengünstige Vertikaleinstelleinheit zur Einstellung einer Vertikalposition des Patiententischs bereitgestellt werden. Insbesondere können derart auch die einzelnen Vertikalpositionen des Patiententischs mit beliebigen Höhen und/oder Positionen in vertikaler Richtung innerhalb eines Einstellbereichs eingestellt werden und sind hierbei nicht an vorgegebene und/oder feste Positionen gebunden.

In einer vorteilhaften Weiterbildung der erfindungsgemäßen Magnetresonanzvorrichtung kann es vorgesehen sein, dass Vertikaleinstelleinheit einen Vertikaleinstellbereich umfasst und die zumindest zwei Vertikalpositionen beliebig innerhalb des Vertikaleinstellbereichs einstellbar sind. Bevorzugt umfasst der Vertikaleinstellbereich für die zumindest zwei Vertikalpositionen eine minimale Vertikalposition und eine maximale Vertikalposition, wobei die zumindest zwei einstellbaren Vertikalpositionen des Patiententischs beliebig zischen der minimalen Vertikalposition und der maximalen Vertikalposition eingestellt werden können. Derart kann eine besonders flexibel und/oder dynamische Einstellung einer Vertikalposition für den Patiententisch erreicht werden.

In einer vorteilhaften Weiterbildung der erfindungsgemäßen Magnetresonanzvorrichtung kann es vorgesehen sein, dass die Patientenlagerungsvorrichtung eine Patiententischsteuereinheit umfasst, die zur Steuerung der Vertikaleinstelleinheit, insbesondere der einzelnen Vertikaleinstellelemente der Vertikaleinstelleinheit, ausgebildet ist. Somit ist die Patiententischsteuereinheit auch zu einer Steuerung einer Positionseinstellung, insbesondere einer Einstellung einer Vertikalposition, des Patiententischs ausgebildet.

Die Patiententischsteuereinheit umfasst zumindest ein Rechenmodul und/oder einen Prozessor. Insbesondere ist die Patiententischsteuereinheit dazu ausgebildet, computerlesbare Instruktionen auszuführen, die in einer Speichereinheit der Patiententischsteuereinheit gespeichert sind. Die Komponenten der Patiententischsteuereinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Mittels der Patiententischsteuereinheit kann eine vorteilhafte Abstimmung der einzelnen Vertikaleinstellelemente zur Einstellung einer Vertikalposition des Patiententischs erfolgen. Insbesondere können derart Vertikaleinstellelemente an der Patientenlagerungsvorrichtung und Vertikaleinstellelemente innerhalb des Patientenaufnahmebereichs vorteilhaft für eine Einstellung einer Vertikalposition des Patiententischs aufeinander abgestimmt werden.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: eine erfindungsgemäße Magnetresonanzvorrichtung mit einer Patientenlagerungsvorrichtung in einer schematischen Darstellung,
- Fig. 2: ein erstes Ausführungsbeispiel einer Vertikaleinstelleinheit mit zumindest einem Vertikaleinstellelement innerhalb eines Patientenaufnahmebereichs der Magnetresonanzvorrichtung,
- Fig. 3: die Magnetresonanzvorrichtung mit dem ersten Ausführungsbeispiel der Vertikaleinstelleinheit in einer Seitenansicht,
- Fig. 4: ein zweites Ausführungsbeispiel einer Vertikaleinstelleinheit mit zumindest einem Vertikaleinstellelement innerhalb eines Patientenaufnahmebereichs der Magnetresonanzvorrichtung,
- Fig. 5: ein drittes Ausführungsbeispiel einer Vertikaleinstelleinheit mit zumindest einem Vertikaleinstellelement innerhalb eines Patientenaufnahmebereichs der Magnetresonanzvorrichtung, und
- Fig. 6: ein Ausführungsbeispiel einer Vertikaleinstelleinheit, die an der Patientenlagerungsvorrichtung angeordnet ist.

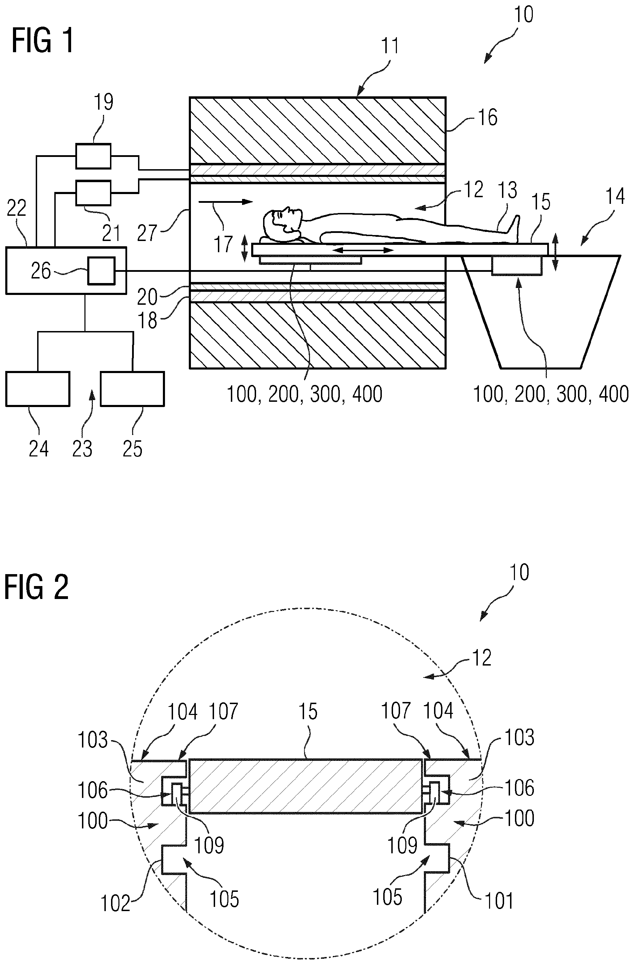

In der Fig. 1 ist eine Magnetresonanzvorrichtung 10 schematisch darstellt. Die Magnetresonanzvorrichtung 10 umfasst eine von einer Magneteinheit gebildeten Scannereinheit 11. Zudem weist die Magnetresonanzvorrichtung 10 einen Patientenaufnahmebereich 12 auf zu einer Aufnahme eines Patienten 13. Der Patientenaufnahmebereich 12 im vorliegenden Ausführungsbeispiel ist zylinderförmig ausgebildet und in einer Umfangsrichtung von der Scannereinheit 11, insbesondere von der Magneteinheit, zylinderförmig umgeben. Grundsätzlich ist jedoch eine davon abweichende Ausbildung des Patientenaufnahmebereichs 12 jederzeit denkbar. Der Patient 13 kann mittels einer Patientenlagerungsvorrichtung 14 der Magnetresonanzvorrichtung 10 in den Patientenaufnahmebereich 12 geschoben und/oder gefahren werden. Die Patientenlagerungsvorrichtung 14 weist hierzu einen innerhalb des Patientenaufnahmebereichs 12 bewegbar ausgestalteten Patiententisch 15 auf. Insbesondere ist hierbei der Patiententisch 15 in Richtung einer Längserstreckung des Patientenaufnahmebereichs 12 und/oder in z-Richtung bewegbar gelagert.

Die Scannereinheit 11, insbesondere die Magneteinheit, umfasst einen supraleitenden Grundmagneten 16 zu einem Erzeugen eines starken und insbesondere konstanten Grundmagnetfelds 17. Weiterhin weist die Scannereinheit 11, insbesondere die Magneteinheit, eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Scannereinheit 11, insbesondere die Magneteinheit, umfasst weiterhin eine Hochfrequenzantenneneinheit 20 zu einer Anregung einer Polarisation, die sich in dem von dem Grundmagneten 16 erzeugten Grundmagnetfeld 17 einstellt. Die Hochfrequenzantenneneinheit 20 wird von einer Hochfrequenzantennensteuereinheit 21 der Magnetresonanzvorrichtung 10 gesteuert und strahlt hochfrequente Magnetresonanzsequenzen in den Patientenaufnahmebereich 12 der Magnetresonanzvorrichtung 10 ein.

Zu einer Steuerung des Grundmagneten 16, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von medizinischen Bilddaten, die während der Magnetresonanzuntersuchung erfasst werden.

Für eine Steuerung einer Bewegung der Patientenlagerungsvorrichtung 14, insbesondere des Patiententischs 15, weist die Patientenlagerungsvorrichtung 14 eine Patiententischsteuereinheit 26 auf. Die Patiententischsteuereinheit 26 ist im vorliegenden Ausführungsbeispiel innerhalb der Systemsteuereinheit 22 der Magnetresonanzvorrichtung 10 integriert. Grundsätzlich kann die Patiententischsteuereinheit 26 auch separat zur Systemsteuereinheit 22 der Magnetresonanzvorrichtung 10 ausgebildet sein.

Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzbilder können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

Für eine Einstellung einer Vertikalposition des Patiententischs 15 weist die Magnetresonanzvorrichtung 10 eine Vertikaleinstelleinheit 30 auf, wobei die Vertikaleinstelleinheit 30 zumindest zwei unterschiedliche Vertikalpositionen für eine Positionierung des Patiententischs 15 innerhalb des Patientenaufnahmebereichs 12 umfasst. In Fig. 1 ist die Vertikaleinstelleinheit 30 nur schematisch dargestellt. Für eine weitere Ausgestaltung der Vertikaleinstelleinheit 30 wird auf die Fig. 2 bis 6 verwiesen.

Die in Fig. 1 dargestellte Magnetresonanzvorrichtung 10 kann selbstverständlich weitere Komponenten umfassen, die Magnetresonanzvorrichtungen 10 gewöhnlich aufweisen. Eine allgemeine Funktionsweise einer Magnetresonanzvorrichtung 10 ist zudem dem Fachmann bekannt, so dass auf eine detaillierte Beschreibung der weiteren Komponenten verzichtet wird.

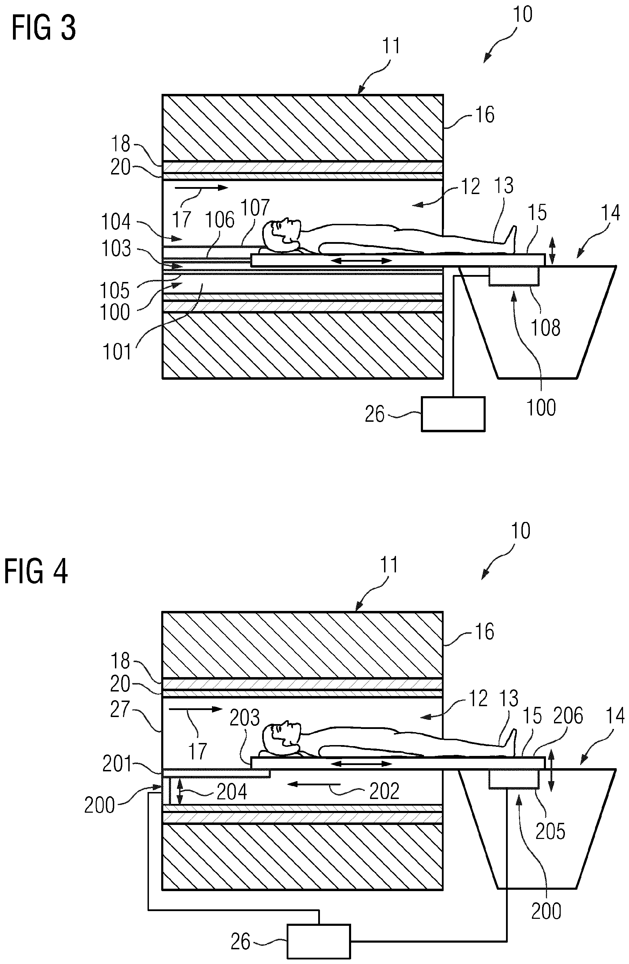

In den Fig. 2 und 3 ist ein ersten Ausführungsbeispiel der Vertikaleinstelleinheit 100 dargestellt. In Fig. 2 ist ein Ausschnitt einer Magnetresonanzvorrichtung 10 dargestellt. Dieser Ausschnitt umfasst einen Querschnitt des Patientenaufnahmebereichs 12. Innerhalb des Patientenaufnahmebereichs 12 ist zumindest ein Vertikaleinstellelement 101, 102 der Vertikaleinstelleinheit 100 angeordnet. Im vorliegenden Ausführungsbeispiel weist die Vertikaleinstelleinheit 100 zwei Vertikaleinstellelemente 101, 102 auf. Die beiden Vertikaleinstellelemente 101, 102 sind jeweils von passiven Vertikaleinstellelementen 101, 102 gebildet. Die beiden Vertikaleinstellelemente 101, 102 umfassen jeweils eine Seitenführung 103, die innerhalb des Patientenaufnahmebereichs 12 angeordnet ist. Die beiden Seitenführungen 103 sind zur Führung des Patiententischs 15 innerhalb des Patientenaufnahmebereichs 12 ausgebildet. Jede der beiden Seitenführungen 103 ist an einem Randbereich 104 des Patientenaufnahmebereichs 12 angeordnet und erstreckt sich in Längsrichtung durch den Patientenaufnahmebereich 12. Die beiden Seitenführungen 103 sind an unterschiedlichen, insbesondere sich gegenüberliegenden Seiten und/oder Randbereichen 104 des Patientenaufnahmebereichs 12 angeordnet sind. Zwischen den beiden Seitenführungen 103 wird der Patiententisch 15 der Patientenlagerungsvorrichtung 14 innerhalb des Patientenaufnahmebereichs 12 geführt.

Vorzugsweise weist jedes der beiden Vertikaleinstellelemente 101, 102, insbesondere jede der beiden Seitenführungen 103, jeweils zumindest zwei unterschiedliche Aufnahmebereiche 105, 106, 107 zur Aufnahme des Patiententischs 15 auf. Im vorliegenden Ausführungsbeispiel weist jedes der beiden Vertikaleinstellelemente 101, 102, insbesondere jede der beiden Seitenführungen 103, drei Aufnahmebereich 105, 106, 107 zur Aufnahme des Patiententischs 15 auf. Die einzelnen Aufnahmebereiche 105, 106, 107 sind jeweils als Führungsschiene ausgebildet. Derart weist die Vertikaleinstelleinheit 100 drei definierte Vertikalpositionen für den Patiententisch 15 auf. In den einzelnen Aufnahmebereichen 105, 106, 107 sind je nach ausgewählter Vertikaleinstellposition Gleitelemente 109 des Patiententischs 15, wie beispielsweise Gleitrollen, in horizontaler Richtung bewegbar gelagert.

Für ein Einbringen des Patiententischs 15 in einen der drei Aufnahmebereiche 105, 106, 107 weist die Vertikaleinstelleinheit 100 ein weiteres Vertikaleinstellelement 108 auf, das an der Patientenlagerungsvorrichtung 14 angeordnet ist, wie dies in Fig. 3, einer Seitenansicht der Magnetresonanzvorrichtung 10 mit der Vertikaleinstelleinheit 100 zu sehen ist. Das weitere Vertikaleinstellelement 108 umfasst ein aktives Vertikaleinstellelement 108, wobei mittels des aktiven Vertikaleinstellelements 108 direkt eine Vertikalposition des Patiententischs 15 aus den drei zur Verfügung stehenden Vertikalpositionen und/oder eine Höhe des Patiententischs 15 eingestellt werden kann. Das aktive Vertikaleinstellelement 108 kann dabei beispielsweise eine Hubschere und/oder weitere, dem Fachmann als sinnvoll erscheinende Vertikaleinstellelemente 108 umfassen.

Eine Steuerung der Vertikaleinstelleinheit 100, insbesondere des aktiven, an der Patientenlagerungsvorrichtung 14 angeordneten Vertikaleinstellelements 108 der Vertikaleinstelleinheit 100, erfolgt mittels der Patiententischsteuereinheit 26 der Patientenlagerungsvorrichtung 14. Hierzu weist die Patiententischsteuereinheit 26 eine Software auf, die bei einem Ausführen durch einen Prozessor der Patiententischsteuereinheit 26 eine Steuerung der Vertikaleinstelleinheit 100 ausführt. Dabei kann ein Benutzer mittels der Benutzerschnittstelle 23 der Magnetresonanzvorrichtung 10 eine Vertikalposition und/oder eine Höhe für den Patiententisch 15 auswählen und die Einstellung der ausgewählten Vertikalposition und/oder Höhe des Patiententischs 15 erfolgt automatisch mittels der Patiententischsteuereinheit 26. Dabei wird von der Patiententischsteuereinheit 26 das aktive Vertikaleinstellelement 108 entsprechend angesteuert. Wird vom Benutzer keine Vertikalposition und/oder Höhe für den Patiententisch 15 ausgewählt, so wird der Patiententisch 15 von der Patiententischsteuereinheit 26 in die mittlere der drei Vertikalpositionen des Patiententischs 15 positioniert, wobei diese mittlere Vertikalposition des Patiententischs 15 ein Grundposition des Patiententischs 15 in vertikaler Richtung umfasst.

Nach einer Einstellung einer Vertikalposition der drei zur Verfügung stehenden Vertikalpositionen des Patiententischs erfolgt ein Einfahren des Patiententischs 15 in den Patientenaufnahmebereich 12, wobei hierbei der Patiententisch 12 abhängig von der eingestellten Höhe und/oder der eingestellten Vertikalposition in einen der drei Aufnahmebereiche 105, 106, 107 der beiden passiven Vertikaleinstellelemente 101, 102 des Patientenaufnahmebereichs 12 eingeführt wird. Das Einfahren des Patiententischs 15 wird ebenfalls von der Patiententischsteuereinheit 26 gesteuert, die hierzu eine nicht näher dargestellte Horizontaleinstelleinheit der Patientenlagerungsvorrichtung 14 ansteuert.

In Fig. 4 ist ein zu den Fig. 2 und 3 alternatives Ausführungsbeispiel der Vertikaleinstelleinheit dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in den Fig. 2 und 3, wobei bezüglich gleichbleibender Bauteile, Merkmale und Funktionen auf die Beschreibung des Ausführungsbeispiels in den Fig. 2 und 3 verwiesen wird.

In Fig. 4 ist die Magnetresonanzvorrichtung 10 mit dem Patiententisch 15 und einer Vertikaleinstelleinheit 200 dargestellt. Die Vertikaleinstelleinheit 200 weist ein Vertikaleinstellelement 201 auf, dass innerhalb des Patientenaufnahmebereichs 12 der Magnetresonanzvorrichtung 10 angeordnet ist. Das Vertikaleinstellelement 201 umfasst hierbei ein passives Vertikaleinstellelement 201, das zu einer Aufnahme des Patiententischs 15 innerhalb des Patientenaufnahmebereichs 12 ausgebildet ist. Das Vertikaleinstellelement 201 ist hierbei an einer rückseitigen Öffnung 27 des Patientenaufnahmebereichs 12 angeordnet und ragt in den Patientenaufnahmebereich 12 hinein. Insbesondere umfasst das Vertikaleinstellelement 201 eine Art Stütze, um den Patiententisch 15 an seinem in Fahrtrichtung 202 weisendem Ende 203 abzustützen.

Der Patiententisch 15 ist hierbei derart ausgebildet, dass ein in den Patientenaufnahmebereich 12 hineinragendes Ende 203 des Patiententischs 15 frei schwebend ist. Das in den Patientenaufnahmebereich 12 hineinragende Ende 203 des Patiententischs 15 umfasst dabei ein in Fahrtrichtung weisendes Ende 203 des Patiententischs 15 während eines Einfahrvorgangs des Patiententischs 15 in den Patientenaufnahmebereich 12. Der Patiententisch 15 ist dabei zunächst nur mit einem dem Patientenaufnahmebereich 12 abgewandten Ende 206 an der Patientenlagerungsvorrichtung 14 abgestützt.

Insbesondere weist der Patiententisch 13 bereit seine definierte Vertikalposition auf, wenn er von dem einem Vertikaleinstellelement 201 innerhalb des Patientenaufnahmebereichs 12 aufgenommen wird. Das innerhalb des Patientenaufnahmebereichs 12 angeordnete Vertikaleinstellelement 201 ist zudem höhenverstellbar ausgebildet, so dass eine Position in vertikaler Richtung 204 des innerhalb des Patientenaufnahmebereichs 12 angeordneten Vertikaleinstellelement 201 einer Vertikalposition des Patiententischs 15 entspricht. Bevorzugt erfolgt die Einstellung und/oder Anpassung einer Position in vertikaler Richtung 204 des innerhalb des Patientenaufnahmebereichs 12 angeordneten Vertikaleinstellelement 201 automatisch mittels der Patiententischsteuereinheit 26. Dabei wird automatisch und/oder selbsttätig von der Patiententischsteuereinheit 26 die Position in vertikaler Richtung 204 des Vertikaleinstellelements 201 innerhalb des Patientenaufnahmebereichs 12 in Abhängigkeit von einer aktuellen und/oder eingestellten Vertikalposition des Patiententischs 15 eingestellt.

Eine Einstellung einer Vertikalposition des Patiententischs 15 erfolgt mittels eines weiteren Vertikaleinstellelements 205 der Vertikaleinstelleinheit 200. Das weitere Vertikaleinstellelement 205 ist an der Patientenlagerungsvorrichtung 14 angeordnet. Das weitere Vertikaleinstellelement 205 umfasst ein aktives Vertikaleinstellelement 295, wobei mittels des aktiven Vertikaleinstellelements 205 an der Patientenlagerungsvorrichtung 14 direkt eine Vertikalpositionen und/oder eine Höhe des Patiententischs 15 eingestellt werden kann. Das aktive Vertikaleinstellelement 205 kann dabei beispielsweise eine Hubschere und/oder weitere, dem Fachmann als sinnvoll erscheinende Vertikaleinstellelemente 205 umfassen. Dabei kann ein Benutzer mittels der Benutzerschnittstelle 23 der Magnetresonanzvorrichtung 10 eine Vertikalposition und/oder eine Höhe für den Patiententisch 15 auswählen und die Einstellung der ausgewählten Vertikalposition und/oder Höhe des Patiententischs 15 erfolgt automatisch mittels der Patiententischsteuereinheit 26 durch ein Ansteuern des aktiven Vertikaleinstellelements 205. Für die Einstellung einer Vertikalposition des Patiententischs 15 weist die Vertikaleinstelleinheit 200 einen Vertikaleinstellbereich mit einer minimalen Vertikalposition und einer maximalen Vertikalposition auf. Die Einstellung einer Vertikalposition des Patiententischs 15 mittels des aktiven Vertikaleinstellelements 205 kann dabei innerhalb des Vertikaleinstellbereichs, insbesondere zwischen der minimalen Vertikaleinstellposition und der maximalen Vertikaleinstellposition, beliebig ausgewählt und/oder eingestellt werden. Insbesondere kann der Benutzer eine beliebige Position innerhalb des Vertikaleinstellbereichs auswählen, die gesteuert von der Patiententischsteuereinheit 26 mittels des aktiven Vertikaleinstellelements 205 eingestellt wird. Wird vom Benutzer keine Vertikalposition und/oder Höhe für den Patiententisch 15 ausgewählt, so wird der Patiententisch 15 von der Patiententischsteuereinheit 26 in eine Grundposition und/oder eine mittlere Vertikalpositionen des Patiententischs 15 positioniert.

Nach einer Einstellung einer Vertikalposition des Patiententischs erfolgt ein Einfahren des Patiententischs 15 in den Patientenaufnahmebereich 12, wobei hierbei das in Fahrtrichtung weisende Ende 203 des Patiententischs 15 freischwebend in den Patientenaufnahmebereich 12 eingebracht wird. Um ein Durchbiegen des Patiententischs 15 zu verhindern, greift das innerhalb des Patientenaufnahmebereichs 12 angeordnete Vertikaleinstellelement 201 unter den Patiententisch 15 zu einer Abstützung. Beispielsweise kann das innerhalb des Patientenaufnahmebereichs 12 angeordnete Vertikaleinstellelement 201 derart ausgebildet sein, dass das Vertikaleinstellelement 201 bereits ab einer Mitte des Patientenaufnahmebereichs 12 unter den Patiententisch 15 greift.

Das Einfahren des Patiententischs 15 wird ebenfalls von der Patiententischsteuereinheit 26 gesteuert, die hierzu eine nicht näher dargestellte Horizontaleinstelleinheit der Patientenlagerungsvorrichtung 14 ansteuert.

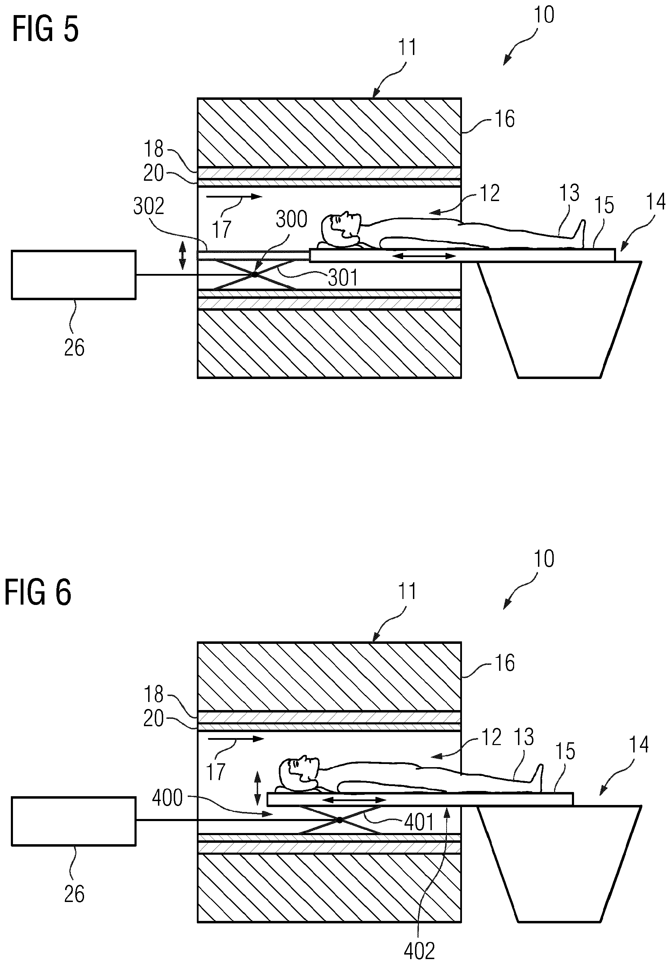

In Fig. 5 ist ein zu den Fig. 2 bis 4 alternatives Ausführungsbeispiel einer Vertikaleinstelleinheit dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu den Ausführungsbeispielen in den Fig. 2 bis 4, wobei bezüglich gleichbleibender Bauteile, Merkmale und Funktionen auf die Beschreibung der Ausführungsbeispiele in den Fig. 2 bis 4 verwiesen wird.

In Fig. 5 ist die Magnetresonanzvorrichtung 10 mit dem Patiententisch 15 und einer Vertikaleinstelleinheit 300 dargestellt. Die Vertikaleinstelleinheit 300 weist ein Vertikaleinstellelement 301 auf, dass innerhalb des Patientenaufnahmebereichs 12 angeordnet ist. Das Vertikaleinstellelement 301 umfasst ein aktives Vertikaleinstellelement 301, wobei mittels des aktiven Vertikaleinstellelements 301 direkt eine Vertikalposition des Patiententischs 15 und/oder eine Höhe des Patiententischs 15 eingestellt werden kann. Im vorliegenden Ausführungsbeispiel weist die Vertikaleinstelleinheit 300 zwei Vertikaleinstellelemente 301 auf, die jeweils von einem aktiven Vertikaleinstellelement 301 gebildet sind, wobei in der schematischen Seitenansicht in Fig. 5 nur eines der beiden Vertikaleinstellelemente 301 zu sehen ist. Jedes der beiden Vertikaleinstellelemente 301 ist an einer Führungsschiene 302 zur Führung des Patiententischs 15 innerhalb des Patientenaufnahmebereichs 12 angeordnet. Die beiden Führungsschienen 302 sind an sich gegenüberliegenden seitlichen Randbereichen innerhalb des Patientenaufnahmebereichs 12 angeordnet.

Die einzelnen Vertikaleinstellelemente 301 sind im vorliegenden Ausführungsbeispiel jeweils als Hubschere ausgebildet, so dass mittels der beiden Vertikaleinstellelemente 301 eine Vertikalposition der Führungsschiene 302 und damit auch den Patiententischs 15 eingestellt werden kann. Hierbei fährt beispielsweise der Patiententisch 15 in einer Grundposition in den Patientenaufnahmebereich 12 ein. Anschließend erfolgt ein Einstellen einer Vertikalposition des Patiententischs 15 mittels der beiden Vertikaleinstellelemente 301, um eine gewünschte Untersuchungsposition des Patiententischs 15 einzustellen. Hierbei werden mittels der beiden Vertikaleinstellelemente 301 die Führungsschienen 302 in die gewünschte Vertikalposition gebracht und damit auch der Patiententisch 15. Aufgrund der Ausbildung der einzelnen Vertikaleinstellelemente 301 als aktive Vertikaleinstellelemente 301, insbesondere als Hubschere, steht für die Einstellung einer Vertikalposition des Patiententischs 15 ein Vertikaleinstellbereich mit einer minimalen Vertikalposition und einer maximalen Vertikalposition der Vertikaleinstelleinheit 300 zur Verfügung. Die Einstellung einer Vertikalposition des Patiententischs 15 mittels der Vertikaleinstellelemente 301 kann dabei innerhalb des Vertikaleinstellbereichs, insbesondere zwischen der minimalen Vertikaleinstellposition und der maximalen Vertikaleinstellposition, beliebig ausgewählt und/oder eingestellt werden. Insbesondere kann der Benutzer eine beliebige Position innerhalb des Vertikaleinstellbereichs auswählen, die gesteuert von der Patiententischsteuereinheit 26 mittels der Vertikaleinstellelemente 301 eingestellt wird.

Dabei kann ein Benutzer mittels der Benutzerschnittstelle 23 der Magnetresonanzvorrichtung 10 eine Vertikalposition und/oder eine Höhe für den Patiententisch 15 auswählen und die Einstellung der ausgewählten Vertikalposition und/oder Höhe des Patiententischs 15 erfolgt automatisch mittels der Patiententischsteuereinheit 26 durch ein Ansteuern der aktiven Vertikaleinstellelemente 301. Wird vom Benutzer keine Vertikalposition und/oder Höhe für den Patiententisch 15 ausgewählt, so wird der Patiententisch 15 von der Patiententischsteuereinheit 26 in eine Grundposition und/oder eine mittlere Vertikalpositionen des Patiententischs 15 positioniert.

Alternativ hierzu können die beiden Vertikaleinstellelemente 301 auch als passive Vertikaleinstellelemente 301 ausgebildet sein. Hierbei kann mittels der beiden Vertikaleinstellelemente 301 die Position der beiden Führungsschienen 302 vor einem Einfahren des Patiententisch 15 auf die gewünschte Vertikalposition des Patiententischs 15 eingestellt werden zur Aufnahme des Patiententisch 15 in dieser Vertikalposition. Hierbei ist der Patiententisch 15 bereits auf die gewünschten Vertikalposition mittels der Vertikaleinstelleinheit 300 und der Patiententischsteuereinheit 26 eingestellt, wie dies in den Fig. 2 bis 4 bereits ausführlich beschrieben ist.

In Fig. 6 ist ein zu den Fig. 2 bis 5 alternatives Ausführungsbeispiel einer Vertikaleinstelleinheit dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu den Ausführungsbeispielen in den Fig. 2 bis 5, wobei bezüglich gleichbleibender Bauteile, Merkmale und Funktionen auf die Beschreibung der Ausführungsbeispiele in den Fig. 2 bis 5 verwiesen wird.

In Fig. 6 ist die Magnetresonanzvorrichtung 10 mit dem Patiententisch 15 und einer Vertikaleinstelleinheit 400 dargestellt. Die Vertikaleinstelleinheit 400 weist ein Vertikaleinstellelement 401 auf, dass an der Patientenlagerungsvorrichtung 14 angeordnet ist. Das Vertikaleinstellelement 401 umfasst ein aktives Vertikaleinstellelement 401, wobei mittels des aktiven Vertikaleinstellelements 401 direkt eine Vertikalposition des Patiententischs 15 und/oder eine Höhe des Patiententischs 15 eingestellt werden kann. Das aktive Vertikaleinstellelement 401 ist an einer Unterseite 402 des Patiententischs 15 angeordnet. Insbesondere umfasst das aktive Vertikaleinstellelement 401 zumindest eine Hubschere, die an der Unterseite 402 des Patiententischs 15 angeordnet ist.

Die Hubschere kann dabei derart angeordnet sein, dass zuerst der Patiententisch 15 in horizontaler Richtung in eine Untersuchungsposition gefahren wird. Hierzu befindet sich der Patiententisch 15 in vertikaler Richtung in der Grundposition. Anschließend wird mittels des Vertikaleinstellelementes 401, insbesondere der Hubschere, in eine vertikale Untersuchungsposition gebracht und/oder gefahren. Zudem kann es auch sein, dass die Hubschere zwischen einer Transporteinheit des Patiententischs 15 und dem Patiententisch 15 angeordnet ist, so dass der Patiententisch 15 auch in einer beliebigen Vertikalposition des Patiententischs 15 in horizontaler Richtung innerhalb des Patientenaufnahmebereich 12 bewegt und/oder verfahren werden kann.

Aufgrund der Ausbildung der einzelnen Vertikaleinstellelemente 401 als aktives Vertikaleinstellelement 401, insbesondere als Hubschere, steht für die Einstellung einer Vertikalposition des Patiententischs 15 ein Vertikaleinstellbereich mit einer minimalen Vertikalposition und einer maximalen Vertikalposition der Vertikaleinstelleinheit 400 zur Verfügung. Die Einstellung einer Vertikalposition des Patiententischs 15 mittels des Vertikaleinstellelements 401 kann dabei innerhalb des Vertikaleinstellbereichs, insbesondere zwischen der minimalen Vertikaleinstellposition und der maximalen Vertikaleinstellposition, beliebig ausgewählt und/oder eingestellt werden. Insbesondere kann der Benutzer eine beliebige Position innerhalb des Vertikaleinstellbereichs auswählen, die gesteuert von der Patiententischsteuereinheit 26 mittels des Vertikaleinstellelements 401 eingestellt wird.

Eine Steuerung des Patiententischs 15, insbesondere eine Einstellung einer Vertikalposition des Patiententischs 15, erfolgt mittels der Patiententischsteuereinheit 26, wie dies in der Beschreibung zu den Fig. 2 bis 4 beschrieben ist.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanzvorrichtung mit einer Scannereinheit, einem von der Scannereinheit zumindest teilweise umgebenen Patientenaufnahmebereich und einer Patientenlagerungsvorrichtung, die einen innerhalb des Patientenaufnahmebereichs bewegbaren Patiententisch aufweist,
**gekennzeichnet durch** eine Vertikaleinstelleinheit zu einer Einstellung einer Vertikalposition des Patiententischs innerhalb des Patientenaufnahmebereichs, wobei die Vertikaleinstelleinheit zumindest zwei unterschiedliche Vertikalpositionen für eine Positionierung des Patiententischs innerhalb des Patientenaufnahmebereiches umfasst.

2. Magnetresonanzvorrichtung nach Anspruch 1,
**dadurch gekennzeichnet, dass** die Vertikaleinstelleinheit zumindest ein Vertikaleinstellelement umfasst, das innerhalb des Patientenaufnahmebereichs angeordnet ist.

3. Magnetresonanzvorrichtung nach Anspruch 2,
**dadurch gekennzeichnet, dass** das zumindest eine Vertikaleinstellelement eine Seitenführung für den Patiententisch mit zumindest zwei unterschiedlichen Aufnahmebereichen zur Aufnahme des Patiententischs umfasst.

4. Magnetresonanzvorrichtung nach einem der Ansprüche 2 bis 3,
**dadurch gekennzeichnet, dass** die Vertikaleinstelleinheit zumindest ein Vertikaleinstellelement umfasst, das an einer rückseitigen Öffnung des Patientenaufnahmebereich zu einer Aufnahme des Patiententischs angeordnet ist.

5. Magnetresonanzvorrichtung nach einem der Ansprüche 2 bis 4,
**dadurch gekennzeichnet, dass** das zumindest eine Vertikaleinstellelement ein aktives Vertikaleinstellelement aufweist zu einer direkten Einstellung einer Vertikalposition des Patiententischs innerhalb des Patientenaufnahmebereichs.

6. Magnetresonanzvorrichtung nach einem der vorhergehenden Ansprüche,
**dadurch gekennzeichnet, dass** die Vertikaleinstelleinheit zumindest ein Vertikaleinstellelement umfasst, das an der Patientenlagerungsvorrichtung angeordnet ist.

7. Magnetresonanzvorrichtung nach Anspruch 6,
**dadurch gekennzeichnet, dass** das zumindest eine Vertikaleinstellelement an einer Unterseite des Patiententischs angeordnet ist.

8. Magnetresonanzvorrichtung nach einem der vorhergehenden Ansprüche,
**dadurch gekennzeichnet, dass** die Vertikaleinstelleinheit zumindest ein Vertikaleinstellelement aufweist, wobei das zumindest eine Vertikaleinstellelement eine Hubschere umfasst.

9. Magnetresonanzvorrichtung nach einem der vorhergehenden Ansprüche,
**dadurch gekennzeichnet, dass** die Vertikaleinstelleinheit einen Vertikaleinstellbereich umfasst und die zumindest zwei Vertikalpositionen beliebig innerhalb des Vertikaleinstellbereichs einstellbar sind.

10. Magnetresonanzvorrichtung nach einem der vorhergehenden Ansprüche,
**dadurch gekennzeichnet, dass** die Patientenlagerungsvorrichtung eine Patiententischsteuereinheit umfasst, die zur Steuerung der Vertikaleinstelleinheit ausgebildet ist.
